# EUROPEAN PATENT APPLICATION

(11) **EP 3 067 042 A1**
(43) Date of publication of application: **14.09.2016**
(21) Application number: 16156258.2
(22) Date of filing: 18.02.2016
(51) Int. Cl.: A61K 9/00, A61K 9/20, A61K 33/10

(54) **FLOATING SYSTEM FOR THE TREATMENT OF GASTRIC DISORDERS**

(30) Priority: 09.03.2015 IT UB20150279
(71) Applicant: S.I.I.T. S.r.l.-Servizio Internazionale Imballaggi Termosaldanti, 20090 Trezzano sul Naviglio MI (IT)
(72) Inventor: MARCELLONI, Luciano, 20090 TREZZANO SUL NAVIGLIO (MI) (IT); SACCHI, Massimo, 20090 TREZZANO SUL NAVIGLIO (MI) (IT); COSTA, Andrea, 20090 TREZZANO SUL NAVIGLIO (MI) (IT)
(74) Representative: Bianchetti Bracco Minoja S.r.l.

(57) **Abstract**

The invention provides a two-layer tablet comprising a) a rapid-disintegration layer containing a salt able to buffer gastric acidity and a mucoadhesive substance that adheres to the gastric mucosa; and b) a floating layer containing a salt able to release carbon dioxide in the presence of acidity, a mucoadhesive substance, and a polymer able to form a hydrogel matrix that retains carbon dioxide after hydration with aqueous fluids. The tablet is useful in the treatment of symptoms of gastric disorders caused by excessive acid secretion and/or reduced functionality of the gastric mucopolysaccharide barrier.

## Description

### Field of invention

The present invention relates to solid oral formulations, in particular a two-layer tablet useful for the differential release of active ingredients used to treat the symptoms of gastric disorders caused by excessive acid secretion and/or reduced functionality of the gastric mucopolysaccharide barrier.

### Background to the invention

One of the most strongly felt problems in the healthcare field today relates to disorders caused by excessive gastric acid secretions and/or reduced functionality of the natural gastric mucopolysaccharide barrier, which generate stomach acidity or hyperacidity.

In the simplest forms of oral administration used to treat the disorders described above, salts able to buffer the pH locally for a short time to lower acidic values, which are therefore less aggressive to the mucosa, are often used.

The gastric pH normally fluctuates between 1.2 and 3.0 units, and salts able to dissolve by buffering excessively acid pH values can be conveniently selected from bicarbonates and citrates, which dissolve at different rates and different pH values; specifically, citrates are more soluble at pH 3, and bicarbonates at pH 1.

The administration forms of said salts often also contain ingredients of natural origin that soothe the mucosa and/or protect it against acid emissions.

Protective ingredients which have been increasingly used recently include polysaccharides, which act by creating a film of mucilage on the irritated mucosa. The barrier effect thus produced prevents ingested or endogenous irritants from coming into direct contact with the tissue.

Various mechanisms for said active ingredients are reported in the literature. They may be (i) associated with the positive charge of polysaccharide residuals, as in the case of chitosan, which gels in an acid medium, or (ii) undergo passive swelling in an acid medium, as in the case of alginates, or (iii) non-specific, as in the case of high-molecular-weight polysaccharides in general, over 18000 Daltons, which are active in forming a barrier that protects the mucosa and tissues against irritant agents.

This latter class includes the polysaccharide Fucoidan, xyloglucans contained in dried tamarind extract, glucomannans and polysaccharides contained in dried extract of Icelandic lichen.

Of all these polysaccharides, both chitosan and fucoidan are known for their properties in the gastric environment, but only chitosan is commonly acknowledged to be mucoadhesive.

In view of the need to solve the problem of excessive acidity rapidly, the most common therapeutic forms for treating disorders due to irritated gastric mucosa are often rapid-release medicaments; the solubilisation of said salts is therefore immediate, or takes a few minutes.

However, this often means that many therapeutic units must be taken at short intervals in view of the extreme dynamics of gastric activity, characterised by the stomach's acid secretions and frequent food filling and voiding cycles.

In general, to ensure that a medicament has a prolonged action, the release of the active constituents can be delayed, often with increased doses, by using the modified-release form, but this assumes that the dose will remain in the site at which it is required to act for the necessary time.

If the gastric pH needs to be buffered and the mucosa protected for a longer time, the problem arises of very fast gastric voiding which prevents the controlled-release form, whether it is the reservoir or matrix type, from fully releasing the salts where they are needed.

Moreover, the therapeutic requirement of a dual action often remains, namely immediate relief from the burning sensation caused by the gastric secretions and maintenance of the effect. A fully controlled-release form would therefore not achieve the first purpose.

A differentiated-release tablet formulation, wherein part of the dose is released immediately and part, which is counteracting to gastric voiding, is released after a longer residence time, would therefore solve the problem, because a slow-release form could encounter problems of early gastric voiding.

### Description of figures

Figure 1 Tablet with two superimposed layers
Figure 2 Maximum mucoadhesive force and work values (Area) obtained by placing the polymer solution (fucoidan) in contact with 0.1M HCl (blank) and with mucin (mean±SD; n=6).
Figure 3 Maximum mucoadhesive force and work values (Area) obtained by placing the hydrated residues of the portion of the tablet remaining after the 30 min disintegration test in contact with 0.1M HCl (blank) and with mucin (mean±SD; n=6).
Figure 4 % adhesion profiles of fluid samples after disintegration at the end of the test (30 minutes) in the presence (mucin) and absence (blank) of mucin (mean±SD; n=3).

### Description of the invention

The present invention offers a solution to the above-mentioned problems by providing a tablet with two superimposed layers for local gastric action, designed to have one layer providing rapid disintegration and release of the antacid and barrier ingredients, and a long-lasting floating layer with mucoadhesive substances that protect the gastric mucosa. The floating layer can conveniently be made with a polymer having characteristics that allow slow release by forming a hydrogel matrix, which is formed after hydration with the aqueous biological fluids.

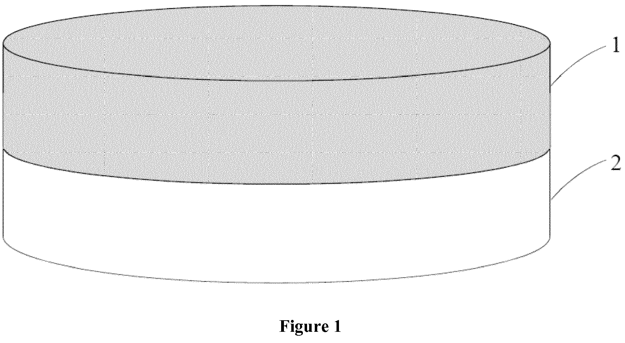

The subject of the present invention is therefore a tablet with two superimposed layers (1,2 - Fig. 1) arranged in such a way that they are simultaneously exposed to the dissolution fluids after swallowing, wherein:
a) a rapid-disintegration layer (1) contains a salt able to buffer gastric acidity and chitosan, with mucoadhesive properties towards the gastric mucosa;
b) a layer that floats after hydration contains a salt able to release carbon dioxide in the presence of acidity, fucoidan, and a polymer able to form a hydrogel matrix after hydration with gastric fluids and trap the carbon dioxide released by bicarbonate, thus generating floating.

The salt able to buffer gastric acidity, preferably with a pH value ranging between 1.2 and 3.0 units, is preferably an alkali or alkaline-earth metal bicarbonate or carbonate or an alkaline citrate, in particular sodium bicarbonate and potassium citrate.

Fucoidan possesses mucoadhesive and soothing properties towards irritated mucosa.

The salt able to release carbon dioxide in the presence of acidity is preferably an alkali or alkaline-earth metal carbonate or bicarbonate, in particular sodium bicarbonate.

Examples of polymers able to form a hydrogel matrix are modified celluloses such as hydroxypropyl methylcellulose (HPMC), in particular Metolose®, hydroxypropylcellulose (HPC), hydroxyethylcellulose (HEC), and carboxyvinyl polymers such as Carbopol®.

The recommended amount of polymer to allow the formation of a hydrogel varies according to the type and grade of polymer, but typically ranges between 5% and 25% by weight.

The tablet according to the invention can optionally contain, in the floating release layer, a salt able to buffer gastric acidity, preferably having a pH value ranging between 1.2 and 3.0 units, for example an alkali metal citrate, in particular potassium citrate.

The rapid-disintegration layer and the floating layer of the tablet according to the invention can also contain excipients and/or additives suitable for pharmaceutical or nutritional use, such as disintegrating agents, binders, lubricants and glidants, sweeteners and flavourings.

In a dissolution test conducted on the preparation, it was found that by using a combination of bicarbonate or another salt able to release carbon dioxide in an acid environnement, medium-molecular-weight hydroxypropyl methylcellulose (HPMC) and high-molecular-weight polysaccharides, after rapid disintegration of the rapid-release layer in gastric fluid with an acid pH, the layer containing hydrogel floats in the stomach in the presence of gastric juices, and said floating is maintained by the polysaccharide residue reduced to mucilage after disintegration of the layer, which typically takes place within 2 hours of gastric residence.

To achieve floating of the layer within 30 minutes of immersion of the form in the gastric fluid, the following amounts have proved effective:
1) Salt able to release carbon dioxide in an acid medium (floating agent): 30 to 190 mg, preferably 85 to 165 mg;
2) Polymer able to form a hydrogel (polymer floating agent): 45 to 135 mg, preferably 60 to 115 mg;
3) Mucoadhesive substance (fucoidan): 50 to 400 mg, preferably 140 to 260 mg.

In a preferred embodiment, the salt able to release carbon dioxide, the polymer and the mucoadhesive substance are present in the floating layer in the amounts of 125 mg, 90 mg and 200 mg respectively.

In order to achieve a floating layer to be deposited effectively on the gastric mucosa after stomach voiding, floating must last for at least 1.5 hours, the average time required for stomach voiding, after the first 30 minutes during which the first layer disintegrates.

This is extremely useful to ensure that the mucoadhesive substance is not removed from the stomach before performing its action, due to the variability in gastric voiding times at different times of day in the same individual, and between different individuals.

The floating can be explained by the presence of bicarbonate in the hydrogel matrix layer which, by releasing gaseous carbon dioxide into an acid medium, generates swelling that produces a great reduction in the density of the layer, until it falls below the density of water, thus causing it to float in the aqueous carrier within 30 minutes of contact with the aqueous gastric fluids.

In the tablets according to the invention, the salt able to release carbon dioxide in an acid medium allows floating, the polymer able to form a hydrogel retains the carbon dioxide released by said salt, and the polysaccharide gives the hydrogel matrix the lightness needed to float.

The two-layer tablet according to the invention produces both rapid and lasting local relief of symptoms caused by excessive gastric acid secretion and/or reduced functionality of the natural gastric mucopolysaccharide barrier. The use of the tablet according to the invention to treat said symptoms represents a further subject of the invention.

The floating material deposited on the walls is mucoadhesive, with sufficient adhesive strength to generate a barrier layer that protects the gastric mucosa.

The two-layer tablet according to the invention maintains its action until gastric voiding, due to the release of salts with a buffer action and polysaccharides with a mucosa-protecting action.

The process for preparation of the two-layer tablet according to the invention is the one commonly used in tablet preparation, employing machines for granulation and drying of the ingredients, mixers and tablet presses, the latter being of a type suitable for the preparation of multi-layer tablets.

The common techniques used to prepare mixtures intended to undergo direct compression or granulation of some of the ingredients (apart from bicarbonate, HPMC and polysaccharide), and subsequent mixing with powder lubricants and glidants, can therefore be conveniently used in the process for preparation of the mixture of ingredients.

The mixing stages are followed by compression in particular rotary tablet presses able to generate tablets with multiple superimposed layers.

Said machines are typically fitted with at least two loading hoppers for the different mixtures corresponding to the different layers, and at least two compression stations, one after the other on the same rotor, where the two different mixtures to be compressed are batched.

In a typical preparation, a first hopper discharges the quantities of the mixture of one of the two layers to be compressed, and the mixture is compressed in a first compression station. The half tablet obtained is then conveyed to the next compression station. There, it is coated by the second granulate batched by the second hopper, which is directly compressed onto the first layer, thus giving rise to the two-layer tablet.

The invention will now be illustrated by the following examples.

### Example 1

| **NAME OF INGREDIENT** | **mg**/**tablet** |
|---|---|
| **Rapid-disintegration layer** | |
| Vegetable CsG chitosan | 300.000 |
| Sodium bicarbonate | 200.000 |
| Red piroxide (E172) (Red iron oxide) | 3.500 |
| Polyplasdone® XL (polyvinylpyrrolidone) | 30.000 |
| Klucel® (hydroxypropylcellulose) | 36.000 |
| Vegetable magnesium stearate | 2.500 |
| Croscarmellose sodium | 43.000 |
| Aerosil® 200 (silicon dioxide) | 5.000 |

| **Floating layer** | |
|---|---|
| Tribasic potassium citrate monohydrate | 50.000 |
| Sodium bicarbonate | 125.000 |
| Fucoidan | 300.000 |
| PVP K30 EP (povidone; polyvinylpyrrolidone) | 10.000 |
| Metolose® 90 SH 4000 (hydroxypropyl methylcellulose) | 88.600 |
| Vegetable magnesium stearate | 3.500 |
| Aerosil® 200 (silicon dioxide) | 2.900 |

### Example 2

| **Rapid-disintegration layer** | **Function** |
|---|---|
| Vegetable CsG chitosan | Polysaccharide /barrier |
| Sodium bicarbonate | Buffer salt for gastric acidity |
| Polyplasdone® XL (polyvinylpyrrolidone) | Channelling disintegrating agent |
| Croscarmellose sodium | Disintegrating agent for swelling |
| Klucel® (hydroxypropylcellulose) | Binder |
| Vegetable magnesium stearate | Lubricant |
| Aerosil® 200 (silicon dioxide) | Glidant |

| **Floating layer** | |
|---|---|
| Sodium bicarbonate | Floating agent which releases carbon dioxide |
| Metolose® 90 SH 4000 (hydroxypropyl methylcellulose) | Polymer floating agent which generates a hydrogel that retains carbon dioxide |
| Fucoidan | Polysaccharide barrier |
| PVP K30 EP (povidone; polyvinylpyrrolidone) | Binder |
| Tribasic potassium citrate monohydrate | Buffer salt |
| Vegetable magnesium stearate | Lubricant |
| Aerosil® 200 (silicon dioxide) | Glidant |

### Example 3

| **NAME OF INGREDIENT** | **mg**/**tablet** |
|---|---|
| **Rapid-disintegration layer** | |
| Vegetable chitosan | 400.000 |
| Red piroxide (E172) (Red iron oxide) | 3.500 |
| Polyplasdone® XL (polyvinylpyrrolidone) | 30.000 |
| Klucel® (hydroxypropylcellulose) | 36.000 |
| Vegetable magnesium stearate | 2.500 |
| Croscarmellose sodium | 43.000 |
| Aerosil® 200 (silicon dioxide) | 5.000 |
| Ginger flavouring | 2.000 |
| Stevia sweetener | 10.000 |

| **Floating layer** | |
|---|---|
| Tribasic potassium citrate monohydrate | 50.000 |
| Sodium bicarbonate | 125.000 |
| Fucoidan | 235.000 |
| PVP K30 EP (povidone; polyvinylpyrrolidone) | 10.000 |
| Metolose® 90 SH 4000 (hydroxypropyl methylcellulose) | 88.600 |
| Vegetable magnesium stearate | 3.500 |
| Aerosil® 200 (silicon dioxide) | 2.900 |
| Stevia sweetener | 10.000 |

### Example 4

### In vitro floating and mucoadhesion study

Measurements were taken to establish the control conditions of the behaviour of the tablet, and in particular of its delayed-release (floating) layer, and to establish the mucoadhesion capacity of the polysaccharide fucoidan it contains.

Preliminary tablet hydration measurements were conducted under disintegration test conditions (700 ml of 0.1M HCl), and the floating portion of 6 tablets containing fucoidan was recovered after 30 minutes' stirring. The recovered portions were weighed to calculate the quantity of fluid absorbed, which amounted to 1.203 g for 6 tablets (200 mg per tablet containing 200 mg of fucoidan).

On the basis of this finding, the fucoidan polymer was hydrated at the rate of 1 g + 1 ml of 0.1M HCl for the mucoadhesion tests. Said tests were conducted by means of traction tests (TAXTPlus Haake tensile tester, equipped with a 1 Kg load cell).

The test was conducted by placing 40 mg of hydrated polymer on the movable probe (diameter 10 mm) and 40 µl of 0.1M HCl (blank) or 40 µl of 8% w/w mucin solution in HCl (porcine gastric mucin) on the support thermostated to 37°C. Each measurement was taken six times.

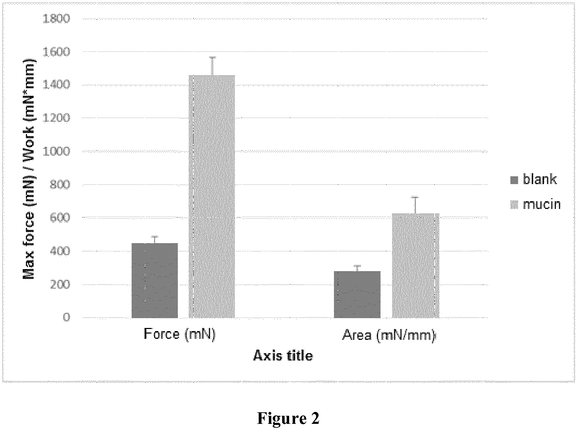

The results are reported in Figure 2, showing both the maximum force at which detachment between the surface of the hydrated polymer and the mucin/buffer took place, and the area under the force/displacement curve (mucoadhesive work). Said figure compares the blank (contact between polymer and HCl only) and mucin (contact between polymer and mucin solution). The increase in adhesive force and adhesive work between blank and mucin represents a positive mucoadhesion index.

A statistical evaluation was conducted between the means with a t test, obtaining a 95% significant difference between mucin and blank for both Maximum Force and Area (Work).

### Mucoadhesion measurements on tablets 30 minutes after start of disintegration test

The test was conducted on 6 two-layer tablets, in a disintegration test apparatus (FU) in 700 ml of 0.1M HCl. All the tablets were collected 30 minutes after the start of the test, and the residues were combined before sampling the aliquots (40 mg) on which the traction test was conducted by TAXTPlus, according to the procedure described above.

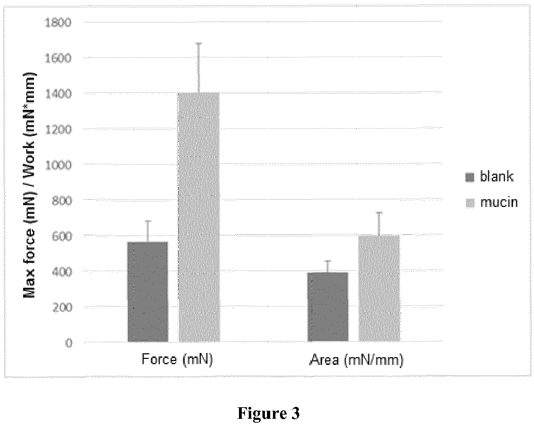

The results are reported in Figure 3, for both the maximum force and the area under the force/displacement (work) curve. Said figure compares the blank (contact between polymer and HCl only) and mucin (contact between polymer and mucin solution). The increase in adhesive force and adhesive work between blank and mucin represents a positive mucoadhesion index.

A statistical evaluation was conducted between the means with a t test, obtaining a 95% significant difference between mucin and blank for both Maximum Force and Area (Work).

An inclined-plane mucoadhesion test was conducted on the disintegration fluid (700 ml of HCl) sampled 30 minutes after the start of the test, containing the dissolved portion of the 6 tablets.

The polysaccharide was confirmed to interact with the mucin of the mucus, demonstrating adhesion.

1 g of sample was placed at the start of the path of a support integral with an inclined plane (inclination 45°) thermostated to 37°C. In the case of the blanks, the support was mucin-free, while in the case of the measurements in the presence of mucin, mucin was spread on the support at the rate of 2.5 ml (conc. 8%) and left to dry so as to form a film before analysis.

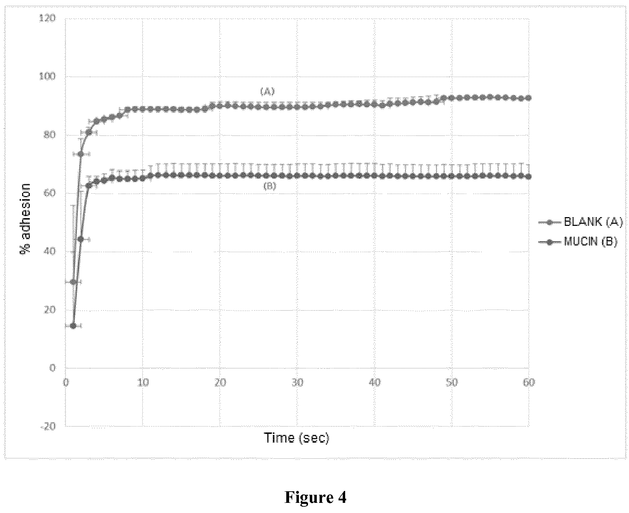

The percentages adhering after 60 seconds (plateau) are shown in the box in Figure 4, wherein the blank represents the adhesion profile without mucin. The results indicate a positive mucoadhesive interaction.

The statistical comparison between the adhesion percentages after 60 seconds in the case of the blanks and the samples was conducted with Student's t test, and a significant difference was found between mucin and blank (P = 0.00033).

## Claims

1. Tablet consisting of two superimposed layers (1, 2), comprising:
a) a rapidly-disintegrating layer containing a salt capable of buffering gastric acidity and chitosan;
b) a layer that floats after hydration, containing a salt capable of releasing carbon dioxide in the presence of gastric acidity, fucoidan, and a polymer capable of forming a hydrogel matrix following hydration with gastric fluids and trapping the carbon dioxide released by bicarbonate, thus generating floating.

2. Tablet according to claim 1 wherein the salt capable of buffering gastric acidity is a carbonate or bicarbonate of an alkali or alkaline-earth metal, or a citrate of an alkali metal, preferably sodium bicarbonate.

3. Tablet according to claim 1 wherein the salt capable of releasing carbon dioxide in the presence of acidity is a carbonate or bicarbonate of an alkali or alkaline-earth metal, in particular sodium bicarbonate.

4. Tablet according to claim 1 wherein the polymer capable of forming a hydrogel matrix is a modified cellulose, preferably selected from hydroxypropyl methylcellulose, hydroxypropyl cellulose and hydroxyethyl cellulose.

5. Tablet according to claims 1-4, wherein the floating layer also contains a salt capable of buffering gastric acidity, preferably an alkali metal citrate, and more preferably potassium citrate.

6. Tablet according to claims 1-5, wherein the floating layer and the rapidly-disintegrating layer contain excipients and/or additives suitable for pharmaceutical use, selected from disintegrating agents, binders, lubricants, glidants, sweeteners and natural flavourings.

7. Tablet according to any one of the preceding claims, consisting of:
a) a rapidly-disintegrating layer comprising sodium bicarbonate and chitosan;
b) a floating layer comprising sodium bicarbonate, fucoidan and hydroxypropyl methylcellulose.

8. Tablet according to claim 7, wherein the floating layer also contains an alkali metal citrate, preferably potassium citrate.

9. Tablet according to any one of the preceding claims wherein, in the floating layer, the salt capable of releasing carbon dioxide in an acid medium is present in amounts ranging from 30 mg to 190 mg, preferably from 85 mg to 165 mg; the polymer capable of forming a hydrogel is present in amounts ranging from 45 mg to 135 mg, preferably from 60 mg to 115 mg; and fucoidan is present in amounts ranging from 50 mg to 400 mg, preferably from 140 mg to 260 mg.

10. Tablet according to claim 9 wherein, in the floating layer, the salt capable of releasing carbon dioxide in an acid medium, the polymer and fucoidan are present in the amounts of 125 mg, 90 mg and 200 mg respectively.

11. Tablet according to claim 9, having the following composition:
| | **mg**/**tablet** |
|---|---|
| **Rapidly-disintegrating layer** | |
| Chitosan | 400.000 |
| Ginger flavouring | 2.000 |
| Red iron oxide | 3.500 |
| Polyvinylpyrrolidone | 30.000 |
| Hydroxypropylcellulose | 36.000 |
| Magnesium stearate | 2.500 |
| Croscarmellose sodium | 43.000 |
| Silicon dioxide | 5.000 |
| Stevia sweetener | 10.000 |
| **Floating layer** | |
|---|---|
| Tribasic potassium citrate monohydrate | 50.000 |
| Sodium bicarbonate | 125.000 |
| Fucoidan | 235.000 |
| Polyvinylpyrrolidone | 10.000 |
| Hydroxypropyl methylcellulose | 88.600 |
| Vegetable magnesium stearate | 3.500 |
| Silicon dioxide | 2.900 |
| Stevia | 10.000 |

12. Tablet according to any one of the preceding claims, for the treatment of gastric disorders due to excessive acid secretion and/or reduced functionality of the gastric mucopolysaccharide barrier.
